(19) Europäisches Patentamt
European Patent Office
Office européen des brevets

(11) Publication number : **0 478 462 A1**

(12) # EUROPEAN PATENT APPLICATION

(21) Application number : **91402592.9**

(22) Date of filing : **27.09.91**

(51) Int. Cl.$^5$ : **C08K 5/524,** C08L 9/00, A61L 29/00

(30) Priority : **27.09.90 JP 258814/90**

(43) Date of publication of application : **01.04.92 Bulletin 92/14**

(84) Designated Contracting States : **BE DE FR GB IT NL SE**

(71) Applicant : **TERUMO KABUSHIKI KAISHA**
**No. 44-1, Hatagaya 2-chome, Shibuya-ku**
**Tokyo 151 (JP)**

(72) Inventor : **Kobayashi, Masahiko, c/o Terumo Kabushiki Kaisha**
**1727-1, Tsuijiarai, Showa-cho**
**Nakakoma-gun, Yamanashi-ken (JP)**

(74) Representative : **Gillard, Marie-Louise et al**
**Cabinet Beau de Loménie 55, Rue d'Amsterdam**
**F-75008 Paris (FR)**

(54) **Radiation resistant polybutadiene composition and medical device containing it.**

(57) A radiation resistant polybutadiene composition, having incorporated in polybutadiene a phosphorous triester containing chain aliphatic groups at least at terminals thereof.

EP 0 478 462 A1

## BACKGROUND OF THE INVENTION

### Field of the Invention:

This invention relates to a radiation resistant polybutadiene composition suitable for medical devices such as medical tubes and medical device member using this composition.

### Description of the Prior Art:

Some of the medical tubes which are used in various medical devices such as tubes for transfusion of blood and other bodily humors, an artificial kidney circuit, and catheters are made of polybutadiene. Polybutadiene is characterized by adsorbing such oil-soluble medical components as nitroglycerin only with difficulty (GB-A-2,153,687). It, however, has the disadvantage that it is colored to yellowish brown when it is subjected to a sterilizing treatment using a radiation such as gamma ray.

As a solution for this disadvantage, a composition having a hindered amine or a salt thereof incorporated in polybutadiene has been known to the art (JP-A-55-19,199(1980)). Further, for the purpose of improving the radiation resistance of such a polyolefin as polypropylene or polybutene, a composition having a hindered amine and a phenol compound or a phosphorous ester compound incorporated in the polyofefin has been proposed (US-A-4,569,736) and US-A-4,507,415). The photostabilizer of hindered amine, however, is short of fully satisfactorily preventing the discoloration.

An object of this invention, therefore, is to provide a novel radiation resistant polybutadiene and medical devices.

Another object of this invention is to provide a radiation resistant polybutadiene composition precluding coloration and dissolution (elution) of the amount of the reducing matter due to exposure to a radiation such as is used for sterilization and enjoying high safety and medical devices which are formed of the composition.

## SUMMARY OF THE INVENTION

These objects are accomplished by a radiation resistant polybutadiene composition having incorporated in polybutadiene a phosphorous triester containing chain aliphatic groups at least at the terminals thereof.

The present invention discloses a radiation resistant polybutadiene composition incorporating therein a phosphonic triester of the structure represented by the formula:

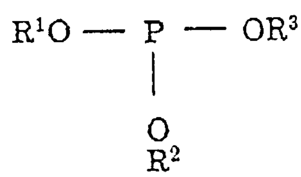

wherein $R^1$ and $R^2$ are independently a saturated or unsaturated chain aliphatic group, $R^3$ is a group of the formula:

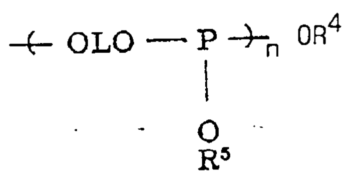

,wherein $R^4$ and $R^5$ are independently a saturated or unsaturated aliphatic group having 3 to 20 carbon atoms, L is an alkylene group, an arylene group, or a group having either or both of said groups linked thereto, and n is for 0 or 1. This invention further discloses a radiation resistant polybutadiene wherein a phosphorous triester is incorporated in polybutadiene in a proportion in the range of from 0.05 to 0.5% by weight, based on the amount of the polybutadiene. This invention also discloses a radiation resistant composition wherein polybutadiene contains a 1,2-bond in a proportion of not less than 80%. This invention discloses a radiation resistant polybutadiene composition satisfying the indicated formula by having 0 for n and the total of carbon atoms of $R^1$, $R^2$, and $R^3$ in the range of from 18 to 54. This invention further discloses a medical device formed of the

radiation resistant polybutadiene composition. This invention also discloses a medical device which has been sterilized by means of radiation. In this specification, radiation resistant means "irradiation-sterilizable."

EXPLANATION OF THE PREFERRED EMBODIMENT

Now, the concrete construction of this invention will be described in detail below.

The base polymer for the construction of the composition of this invention is polybutadiene. Particularly, the polybutadiene is preferable to be 1,2-polybutadiene. The 1,2-polybutadiene is preferable to be syndiotactic in form.

Though the base polymer may be formed solely of the syndiotactic 1,2-polybutadiene, it may be formed of a mixture thereof with an isotactic 1,2-polybutadiene or with a trans form or cis form 1,4-polybutadiene. In this case, the polybutadiene base polymer is preferable to contain not less than 80%, preferably 90 to 100%, of a 1,2-bond. The degree of polymerization of this polybutadiene base polymer, as determined by the X-ray method, is preferably to be in the range of 10 to 50%, preferably 15 to 30%. Though the present invention does not particularly discriminate the polybutadiene on account of its molecular weight, it suffices to use polybutadiene whose number average molecular weight is approximately in the range of from 50 000 to 300 000, preferably from 100,000 to 150,000. Further, the distribution of this molecular weight has no particular restriction. The density of the butadiene is generally sufficient approximately in the range of from 0.895 to 0.910.

The polybutadiene base polymer of the quality described above incorporates therein a phosphorous triester (triester of phosphorous acid). The phosphorous triester to be used herein is a monovalent phosphorous triester having chain aliphatic groups at least at the terminals thereof. To be specific, it is a phosphorous tri-aliphatic ester whose three substituents are saturated or unsaturated chain aliphatic groups having 3 to 20 carbon atoms, preferably 6 to 18 carbon atoms. Optionally, the aforementioned monovalent aliphatic groups may be substituted for the terminals of part or whole of the aliphatic groups of the aforementioned phosphorous tri-aliphatic ester.

The phosphorous triester described above is preferably to have a structure represented by the following formula:

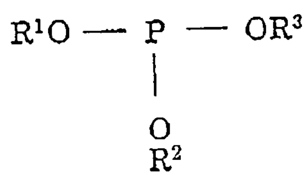

wherein $R^1$ and $R^2$ are independently a saturated or unsaturated chain aliphatic group having 3 to 20 carbon atoms, preferably 6 to 18 carbon atoms, $R^3$ is a group of the formula:

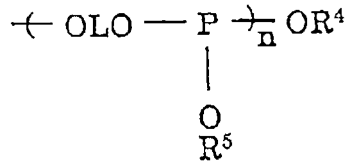

wherein $R^4$ and $R^5$ are independently a saturated or unsaturated chain aliphatic group having 3 to 20 carbon atoms, preferably 6 to 18 carbon atoms, L is an alkylene group, particularly an alkylene group having 3 to 30 carbon atoms, preferably 6 to 18 carbon atoms, an arylene group, particularly an arylene group having 6 to 40 carbon atoms, preferably 8 to 25 carbon atoms, or a group combining these groups such as, for example, an alkylene-arylene group or an arylene-alkylene-arylene group, and n stands for 0 or 1.

$R^1$, $R^2$, $R^4$, and $R^5$ are equally or unequally for chain aliphatic groups as described above, which chain aliphatic groups may, or may not, be possessed of a branch. Where n is 0, the total number of carbon atoms of $R^1$, $R^2$, and $R^4$ is in the range of from 18 to 54, preferably from 24 to 54. Where n is 1, the total number of carbon atoms of $R^1$, $R^2$, $R^4$, and $R^5$ is in the range of from 8 to 80, preferably from 18 to 54. Incidentally, n is preferable to be 0.

The phosphorous triester, as described above, can be synthesized by a known method. Such commercialy available phosphorous triesters as Chelex 0 and OL made by Sakai Chemical Industry Co., Ltd. and MARK

1500 and 260 made by Adeka-Argus Chemical Co., Ltd. may be adopted in their unmodified form.

Typical examples of the phosphorous triester to be used in the present invention will be cited below. The phosphorous triester for use in this invention is not limited to these typical examples.

$(C_6H_{13}O)_3P$

$(C_8H_{17}O)_3P$

$(C_{10}H_{21}O)_3P$

$(C_{12}H_{25}O)_3P$

$(C_{14}H_{29}O)_3P$

$(C_{16}H_{33}O)_3P$

$(C_{16}H_{31}O)_3P$

$(C_{18}H_{37}O)_3P$

$(C_{18}H_{35}O)_3P$

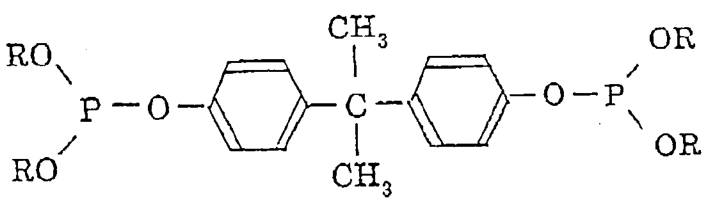

(R=C12~C15 alkyl)

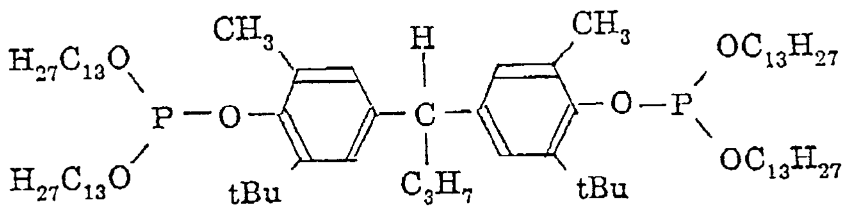

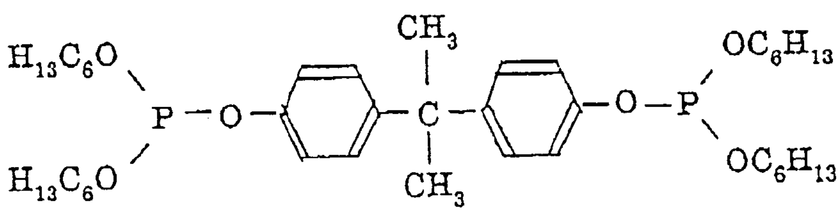

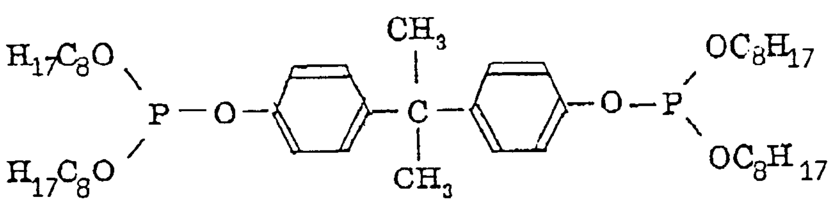

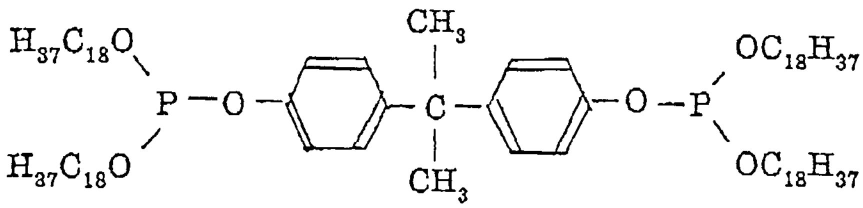

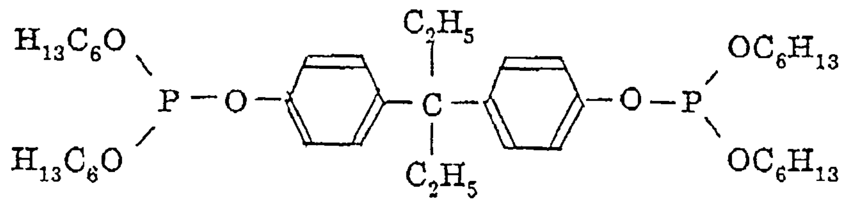

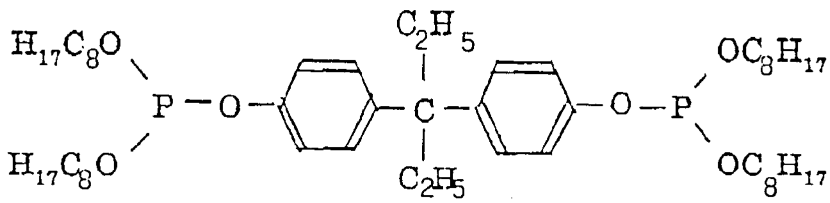

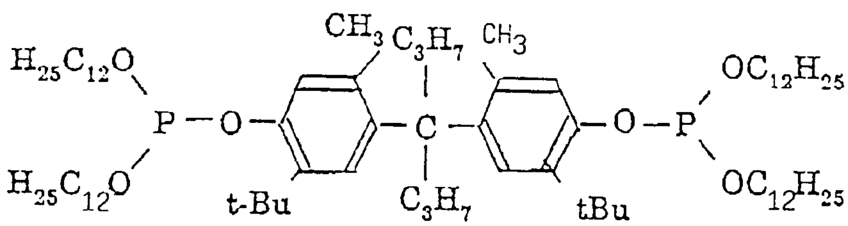

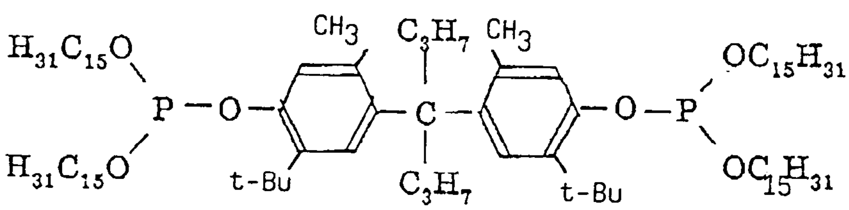

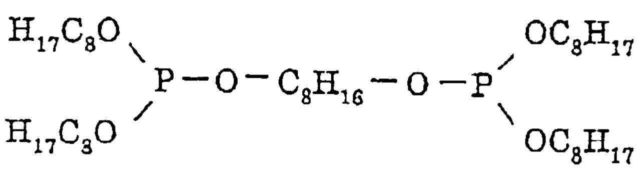

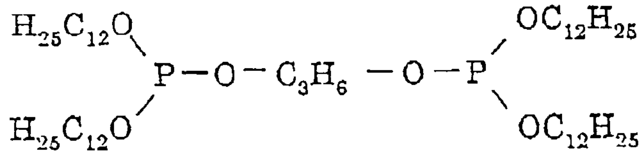

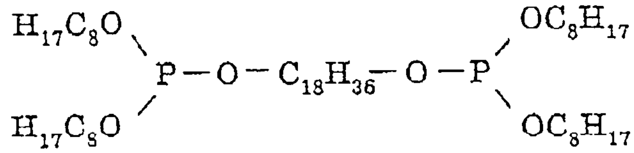

One or more members selected from the group of phosphinic triesters cited above are incorporated in the polybutadiene base polymer in a proportion in the range of from 0.05 to 0.5% by weight, preferably from 0.1 to 0.4 % by weight, based on the weight of the base polymer.

The composition having the phosphinic triester incorporated in polybutadiene may additionally contain therein a slip additive such as paraffin wax or fatty acid amide in a proportion of not more than 1.0% by weight, based on the amount of the base polymer.

The composition is molded in the form of a varying medical tool such as a bag or a tube. The form in which the composition is molded may be varied. When the product of molding is a tube, the initial modulus of elasticity G is preferable to be not more than 5 $kgf/mm^2$. The magnitude of this initial modulus of elasticity G is computed of the leading edge part of the range of minimum amount of change in the deformation-stress curve in accordance with the formula, $G = W \cdot \ell / \Delta\ell \cdot A$ [wherein W is an applied load (kg), $\ell$ for the initial length (mm), $\Delta\ell$ is elongation (mm), and A is a cross section ($mm^2$)], as specified in JIS (Japanese Industrial Standard) K 7113.

When the initial modulus of elasticity is not more than 5 $kgf/mm^2$, the tube enjoys notably improved flexibility, suffers less possibility of sustaining a bend, and exhibits improved resiliency. The tube also shows improvement in low temperature properties, particularly in flexibility and impact resistance at low temperatures. From the viewpoint of flexibility, it is ideal for the initial modulus of elasticity to be in the range of from 0.1 to 4.0 $kgf/mm^2$.

Further, in terms of transparency, the absorbance of the tube at 500 nm per mm of thickness is desired to be in the range of from 0.18 to 0.65.

The molding of the composition can be carried out by extrusion molding as conventionally practised. The work of molding, therefore, is very easy and inexpensive. In this case, the production of the molded medical tool is facilitated by having the composition dry blended with the screw in an extrusion molding device in use. The tube which is molded as described above generally has an inside diameter approximately in the range of from 1 to 20 mm and a wall thickness approximately in the range of from 0.3 to 3.5 mm. The tube, with the dimensions in the ranges mentioned above, exhibits extremely preferable flexibility. The cross-sectional shape, length, etc. of this tube are variable in a wide range.

The medical tubes of this kind are used as connecting tubes for bodily humor-transfusing sets and blood-transfusing sets, as tubes for transporting bodily humors or blood through various extra-corporal circulation paths in dialysers and artificial kidneys, as tubes for various stationary needles, as various catheters, and as tubes for respiratin circuits.

These various medical tools are assembled by joining the medical tubes to other components parts in stated layouts by high-frequency fusion or subjecting the medical tubes to other desired secondary fabrications. The medical tools thus assembled or additionally fabricated are subjected to a sterilizing treatment with radiation such as gamma ray or electron beam. The dosage of the radiation for this treatment is in the range of from 0.5 to 5 Mrads, preferably from 1.0 to 3.5 Mrads.

Now, the present invention will be described more specifically below with reference to working examples.

Example 1

Syndiotactic 1,2-polybutadiene having an average molecular weight of 130,000, a crystallization degree of 20%, and a 1,2-bond content of 90% (produced by Japan Synthetic Rubber Co., Ltd. and marketed under product code of "RB810") was adopted as a base polymer. A mixture of 100 parts by weight of this base polymer

and 0.05 part by weight of paraffin wax (produced by Nippon Seiro K.K. and marketed under trademark designation of "Luvax 2191") was introduced into a hopper of an extrusion molding device, blended inside a screw cylinder of the extrusion molding device at 130°C, and extruded through a die thereof at 110°C to give rise to a continuous tube No. 1 having an inside diameter of 2.1 mm and an outside diameter of 3.3 mm.

Separately, aliquots of the same composition as mentioned above severally containing the following stabilizers, 1 to 4, and a hindered amine type stabilizer in a fixed ratio of 0.3 part by weight, based on 100 parts by weight of the base polymer were extrusion molded by the procedure described above, to produce tubes, Nos. 2 to 6, of the same size as mentioned above.

Stabilizer 1: Chelex O produced by Sakai Chemical Co., Ltd.

$$(C_8H_{17}O)_3P$$

Stabilizer 2: Chelex OL produced by Sakai Chemical Co., Ltd.

$$(C_8H_{35}O)_3P$$

Stabilizer 3: MARK 1500 produced by Adeka-Argus Chem. Co., Ltd.

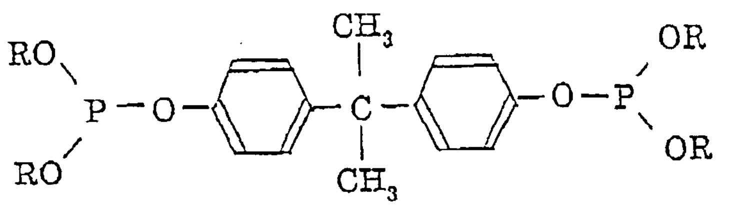

(R=C12~C15 alkyl)

Stabilizer 4: MARK 260 produced by Adeka-Argus Chem. Co., Ltd.

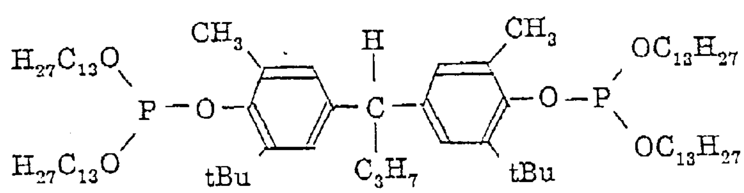

Hindered amine type stabilizer: MARK LA 63 produced by Adeka-Argus Chem. Co., Ltd.

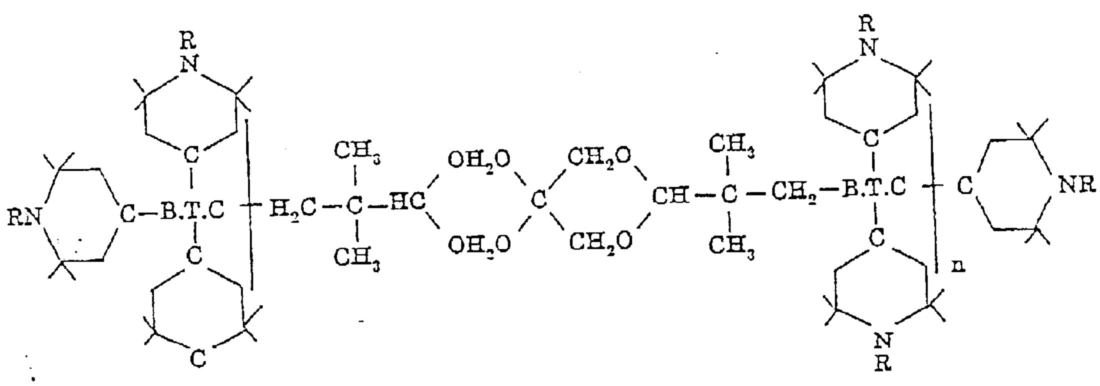

R : CH3 B.T.C : 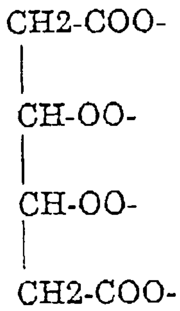 molecular weight about 2,000

These six tubes were sterilized by six hours exposure to gamma ray at a dose 2.0 Mrads. The amounts of discoloration caused on the tubes in consequence of the sterilization were determined by measuring the b values with a color meter. The results are shown in Table 1.

The six tubes were then tested for dissolved matter. A 15-g sample from a given tube was cut into minute pieces, boiled in about 150 ml of water for 30 minutes, and diluted with water to a total volume of 150 ml. The resultant solution was used as a test liquid. Ten (10) ml of the test liquid and 20 ml of a 0.01N potassium permanganate solution and 1.0 ml of dilute sulfuric acid were jointly boiled for three minutes and then cooled. The resultant liquid was combined with 0.1 g of potassium iodide and five drops of starch reagent and titrated with a 0.01N sodium thiosulfate solution. The amounts of reducing matter dissolved out ($\Delta KMnO_4$) indicated by the differences of the amounts of the potassium permanganate solution consumed from the amount found in a blank test are shown in Table 1.

The test liquids and KCl added thereto were subjected to determintion of pH in accordance with the procedure specified by the Japanese Pharmacopoeia. The differences ($\Delta$ pH) from the blank are shown in Table 1.

Table 1

| Tube | Stabilizer | Coloraion b value | | △KMnO4 (mℓ) | △pH |
|---|---|---|---|---|---|
| | | No radiation | After γ ray radiation | | |
| 1 (Control) | — | 1.9 | 7.5 | 0.22 | 0.20 |
| 2 | 1 | 1.9 | 2.0 | 0.31 | 0.21 |
| 3 | 2 | 1.9 | 2.2 | 0.30 | 0.25 |
| 4 | 3 | 1.9 | 3.0 | 0.28 | 0.30 |
| 5 | 4 | 1.9 | 3.1 | 0.31 | 0.28 |
| 6 (Control) | Control | 1.9 | 3.2 | 0.84 | 0.27 |

It is clearly noted from the results given in Table 1 that the tubes produced from the compositions of the present invention were discolored very little by exposure to radiation and possessed of high safety. The amounts of reducing matter of the tube produced from the composition of the present invention were dissolved out very little.

## Claims

1. A radiation resistant polybutadiene composition, having incorporated in polybutadiene a phosphorous triester containing chain aliphatic groups at least at the terminals thereof.

2. A composition according to claim 1, wherein said phosphoric triester is possessed of a structure represented by the following formula:

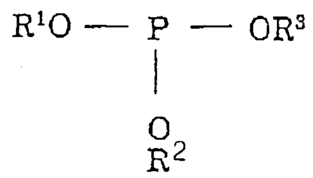

wherein $R^1$ and $R^2$ are independently a saturated or unsaturated chain aliphatic group having 3 to 20 carbon atoms and $R^3$ is a group of the formula:

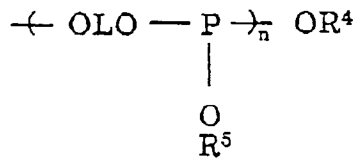

wherein $R^4$ and $R^5$ are independently a saturated or unsaturated chain aliphatic group having 3 to 20 carbon atoms, L is an alkylene group, an arylene group, or a group having either or both of said groups bound thereto, and n stands for 0 or 1.

3. A composition according to claim 1, wherein said phosphorous triester is incorporated in polybutadiene in a proportion in a range of from 0.05 to 0.5% by weight, based on the amount of said polybutadiene.

4. A composition according to claim 1, wherein said polybutadiene contains a 1,2-bond in a proportion of not less than 80%.

5. A composition according to claim 1, wherein said polybutadiene contains a 1,2-bond in a proportion in a range of from 90 to 100%.

6. A composition according to claim 2, wherein $R^1$, $R^2$, $R^4$, and $R^5$ are saturated or unsaturated chain aliphatic groups having 6 to 18 carbon atoms.

7. A composition according to claim 2, wherein n is 0 and the total number of carbon atoms of $R^1$, $R^2$, and $R^4$ is in the range of from 18 to 54.

8. A composition according to claim 6, wherein n is 1 and L is an arylene-alkylene-arylene group.

9. A medical tool, formed of a radiation resistant polybutadiene composition set forth in any of claims 1 to 8.

10. A medical tool according to claim 9, which has been sterilized by means of radiation.

European Patent Office

# EUROPEAN SEARCH REPORT

Application Number

EP 91 40 2592

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | US-A-3 822 225 (D.V.BRADDON ET AL.)<br>* claim 1 * | 1-3,6-7 | C08K5/524<br>C08L9/00<br>A61L29/00 |
| X | CHEMICAL ABSTRACTS, vol. 71, no. 24, Columbus, Ohio, US;<br>abstract no. 113949, '& JP-A-44008747 (ASAHI CHEM CO) 23-04-1969'<br>* abstract * | 1-3,6-7 | |
| X | WORLD PATENTS INDEX LATEST<br>Week 8104,<br>Derwent Publications Ltd., London, GB;<br>AN 05119D<br>& SU-A-734229 (RATNIKOVA ET AL.) 16-05-1980<br>* abstract * | 1-3,6-7 | |
| A | WORLD PATENTS INDEX LATEST<br>Week 8549,<br>Derwent Publications Ltd., London, GB;<br>AN 307801<br>& JP-A-60215036 (MITSUI PETROCHEM) 28-10-1985<br>* abstract * | 9-10 | **TECHNICAL FIELDS SEARCHED (Int. Cl.5 )** |
| D,A | GB-A-2 153 687 (SMITH AND NEPHEW ASSOCIATED COMPANIES PLC.) | 4-5,9-10 | C08K<br>A61L |
| A | * page 1, line 42 - page 1, line 43; claim 2 * | 9-10 | |
| A | GB-A-1 180 398 (PURE CHEMICALS LIMITED)<br>* claim 8 * | 8 | |

The present search report has been drawn up for all claims

| Place of search | Date of completion of the search | Examiner |
|---|---|---|
| THE HAGUE | 13 JANUARY 1992 | VAN HUMBEECK F. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P0401)